## Europäisches Patentamt
## European Patent Office
## Office européen des brevets

(19)

(11) Publication number: **0 057 877**

**B1**

(12) **EUROPEAN PATENT SPECIFICATION**

(45) Date of publication of patent specification: **10.07.85**

(51) Int. Cl.⁴: **A 61 N 1/04**

(21) Application number: **82100642.6**

(22) Date of filing: **01.02.82**

(54) Implantable medical lead.

(30) Priority: **02.02.81 US 230508**

(43) Date of publication of application:
**18.08.82 Bulletin 82/33**

(45) Publication of the grant of the patent:
**10.07.85 Bulletin 85/28**

(84) Designated Contracting States:
**DE FR GB NL**

(56) References cited:
**DE-A-3 006 472**
**US-A-3 476 116**
**US-A-4 230 604**

(73) Proprietor: **MEDTRONIC, INC.**
**3055 Old Highway Eight**
**Minneapolis Minnesota 55440 (US)**

(72) Inventor: **Juncker, David F.**
**16 E Minnehaha Parkway**
**Minneapolis, Minn. 55427 (US)**
Inventor: **Williams, Terrell M.**
**1444 97th Avenue N.W.**
**Coon Rapids, Minn. 55433 (US)**

(74) Representative: **Schwan, Gerhard, Dipl.-Ing.**
**Elfenstrasse 32**
**D-8000 München 83 (DE)**

Courier Press, Leamington Spa, England.

EP 0 057 877 B1

## Description

Electrical stimulation of the body for medical purposes is well known in the prior art. An example of a device for this purpose is the cardiac pacemaker. In the pacemaker context, as well as other body stimulation contexts, the stimulation is delivered to the desired body site by an electrode component of a lead.

A variety of body implantable electrical leads for the delivery of electrical stimulation energy to a desired body site are also known in the prior art. Recent years have seen increased usage of transvenous leads which can deliver the stimulation energy to the desired body site while significantly lowering patient risk and morbidity. For example, the use of a transvenous/endocardial lead eliminates the necessity of a thoracotomy while delivering stimulation energy to the heart to provide a pacemaking function.

In many stimulation contexts, maintenance of a reliable electrical contact at the desired electrode site has proven difficult. Interactions between the lead and the contact site can vitiate the desired effects of the stimulation. For example, material reactions may encourage fibrosis. In the pacemaking context, fibrosis is believed to be a major factor in the increase in chronic threshold that is usually experienced. Also, mechanical trauma may result in inflammation of the tissue to be stimulated. Such inflammation may alter the response of the tissue to the stimulation energy, both acutely and chronically.

In the chronic use of such leads, it has been observed that the contractions of the heart against the lead and the mechanical pressure applied against the heart by the lead may traumatize the contacted tissue. The traumatized tissue may form scar tissue around the electrode(s). In chronic experience, it is observed that the threshold currents sufficient to stimulate the heart increase as the scar tissue is formed until the thickness of the scar tissue stabilizes. The required energy to stimulate the heart is thereby increased because of the additional potential drop across the nonresponsive cardiac cells. A discussion of this mechanism in conjunction with the realization of optimum electrode surface area appears in the paper entitled "Comparison of Power Sources for Advanced Pacemaker Applications" by Rasor, Spickler and Clabaugh, *Proceedings of the Seventh Intersociety Energy Conversion Engineering Conference*, January 1972, pages 752—760.

Pressure induced trauma are believed to be the leading cause of enlarged fibrotic encapsulation of implanted electrode tips.

In addition, cardiac tissue is occluded under the tip and deprived of access to oxygen and water soluble nutrients carried by the blood while also being deprived of the ability to transfer waste products from the tissue into the blood. A large number of tissue cells are so occluded by existing endocardial electrode tips because cells of heart tissue must communicate with the blood in the ventricles of the heart by way of diffusion due to the lack of capillaries near the endocardial surface.

The interactions noted above have long been recognized and efforts have been made to ameliorate their consequences. For example, leads have been configured to reduce mechanical trauma and the response of irritable tissue during lead placement. Materials have been selected for the lead body and electrodes to minimize fibrosis. Thrombus formation may also be countered by the administration of suitable drugs.

The administration of drugs to counter the undesirable interactions between the lead and body noted above has not gained widespread acceptance in that it has heretofore required a systemic treatment to counter a localized interaction. Also, lead configuration must take into account other factors such as focus of sufficient current density into viable, depolarizable tissue, the efficiency of the delivery of the stimulation energy, the ease of lead placement, maintenance of the desired electrode position and reliability of the lead over extended periods of time. An accommodation of these interests has resulted in leads whose configuration necessarily results in undesirable interactions between the lead and body.

Other interactions, such as electromechanical and polarization effects, between the electrode and body at the contact site, can result in the occurrence of undesirable events, and waste energy stored in pacemaker batteries. In some circumstances where electrical stimulation of body tissue is indicated, the tissue to be stimulated is irritable. The placement of a pacemaking lead may induce a cardiac arrhythmia. The presence of the lead may also promote thrombus formation.

The behavior of the metals employed as stimulation electrodes in implantable leads has long been of considerable interest, particularly in such applications as cardiac pacemakers. In most commercial pacemaker leads, the electrodes are of platinum or a platinum alloy. The lead is inserted into the heart tissue or into the right ventricle by transvenous catheter. The energy required to stimulate the heart depends on the particular electrode use. For any given stimulation electrode, the electrical behavior of the tissue around it and the effect of the electrode on the tissue can be evaluated from the stimulation threshold and the operational value of the electrode circuit. The term "threshold" means the smallest amount of electrical energy which will produce consistent cardiac contraction with the electrode system used when the stimulation impulse is delivered after the absolute and the relative refractory period of the heart.

Typically, the threshold increases during the first weeks of implantation caused by interstitial edema and the growth of connecting tissue around the lead electrode tip. With metallic electrodes the stimulation energy cannot be substantially reduced by changing the surface area of the electrode or the impulse duration.

Since high current density and a small electrode is required locally to stimulate the cardiac muscle, polarization effects develop with the consequence that most of the delivered energy from the pacemaker circuit is wasted in the electrode electrolyte interface, a region which is termed the Helmholtz layer. Polarization losses are particularly important in the case of implanted pacemakers since their life time is determined by the amount of energy stored in their batteries. Considerable improvement of the system life time can be achieved if the stimulation system is used more economically.

In US—A—3 476 116 there is described a lead with means for increasing the current density at the electrode contact.

It is recognized that ideally the processes taking place at electrode contact surfaces should be solely those involving motion of electric charges by ion carriers without polarization effects. Unfortunately, the requirement for high current density is ordinarily accompanied by high polarization effects. In implantable leads, polarization effects exist in the vicinity of the Helmholtz layer wherein the interchange from the electron flow of the metal electrode to the ionic flow of the electrolyte (body fluids) occurs.

Parsonnet et al. described a non-polarizing electrode in "Clinical Use Of A New Transvenous Electrode", *Ann. N.Y. Acad. Sci.*, 167:756, 1969. In this system, termed a "Differential Current Density Electrode" the waste of energy due to polarization effects was reduced by providing a low current density at the metal electrode to avoid polarization and a high current density at the point of contact to the endocardium. This system provided lowered stimulation thresholds but suffered from electrochemical instability during long-term operation.

Another type, a purely capacitive electrode, was described by Schaldock in an article entitled "New Pacemaker Electrodes", *Transactions: American Society Artificial Internal Organs*, Volume 29, pages 39—35, 1971.

The present invention provides an implantable lead comprising a conductive polymer gel electrode contacted over a relatively large part of its surface by a metal contact electrode and having a relatively small part of its surface area exposed for contacting body tissue, the metal contact electrode and the polymer gel electrode, except for the tissue-contacting surface thereof, being enclosed against contact with tissue and body fluids by an insulating sheath, and an electrical conductor connected to the metal contact electrode.

The polymer gel electrode constitutes a non-metallic replacement electrode for the noble metal or other metal electrodes used heretofore in implantable leads. The conductive polymer-based gel, preferably a hydrogel, may be cast to shape or formed in situ. The polymer-based gel electrode, due to its configuration, avoids the polarization phenomena associated with metal electrodes in a conductive fluid as is encountered in living bodies. The electrode of the invention provides a transition zone where electron flow is converted to ionic flow i.e., the Helmholtz layer. With metal electrodes, the Helmholtz layer is external to the electrode and is such that cells in its vicinity are disrupted or otherwise damaged. With the polymer-based gel electrode of the invention a transitional synthetic layer is provided in the electrode where this reaction can take place.

Significant advantages of the improved implantable lead of the invention are: reduced tissue trauma; use of inexpensive gel electrode rather than expensive precious metal electrodes; improved electrode-tissue interface, mechanically, chemically and electrically; use of non-corroding electrode material, and protection of the living cells from any electron/ion conversion reaction.

Most significantly, however, the polymer-based gel electrode of the implantable lead of this invention is provided with a relatively small surface area and high ion current density for tissue contact. The polymer-based gel body provides a volume in which the electric charge flow interchange occurs rather than in the body fluids (electrolyte) whereby polarization effects occur in the electrode proper and not in the fluids at the tissue contact area. By providing a relatively large surface area of polymer gel body for contacting the conductive lead means and providing electron flow to the gel electrode body i.e., large relative to the stimulating surface area of the gel electrode, it is possible to provide for high interchange of electron to ion flow with relatively low impedance while providing high current density at the relatively small stimulating, tissue contact surface of the gel electrode.

Small surface area at the stimulating portion of the gel electrode may be controlled by aperture size in the lead body and by gel body size at the exposed contact portion of the gel body and by various combinations of these two aspects.

The polymer gel electrode which is used in the lead of the present invention and which ameliorates the effects of undesirable interactions between the lead and the contacted tissue, constitutes a body of soft, compliant, water swellable, polymeric, water insoluble material non-toxic to body tissues and fluids. The material preferably consists essentially of a hydrogel which is ionically conductive. The hydrogel is permeable to water, oxygen, $CO_2$ and the like. Consequently, their diffusion may occur through the cushioning body both to and away from the occluded tissue cells at the contact site. The water permeable hydrogel body is ionically conductive thereby providing electrical contact between the metal electrode and the tissue.

Embodiments of the present invention are described in detail below with reference to the drawings, in which:

Fig. 1 illustrates a portion of a body implantable lead having a polymer gel electrode in accordance with a preferred embodiment of the present invention;

Fig. 2 is an end view of the body implantable lead shown in Fig. 1;

Figs. 3 and 4 are fragmentary showings of the distal end of implantable leads having differing polymer gel electrode configurations according to the invention.

The concept of using a conductive polymer gel as the stimulating and sensing medium in an implantable transvenous lead, particularly in a cardiac pacing lead, arose from considerations of the electrochemistry at the electrode/tissue (electrolyte) interface. The charge carriers in metal and similar electrical conductors are electrons whereas charge is carried by ions in electrolyte solutions. When metal is immersed in an electrolyte, charge flows from one phase to another until the electrochemical potentials of the metal and electrolyte phases are equal. This results in the buildup of a layer of charge, called the Helmholtz layer, around the surface of the electrode. This phenomenon is termed polarization and results in a dissipative energy loss that shortens pulse generator battery life. Electrochemical reactions may also result in corrosion of the electrode metal, depending on the metal used.

In addition to the detrimental effects of the Helmholtz layer on device operation, there is some question as to whether the chronic presence of a charge layer near the tissue causes myocardial and endocardial pathologic processes. For example, the Helmholtz layer may chronically alter the local pH in the vicinity of the electrode. The chronic imbalance of charge may affect the membrane properties such that changes in myocardial and endocardial cell depolarization threshold occur.

Because of the known, as well as the unknown or speculative, effects attributable to the Helmholtz layer, the gel electrode of the invention is proposed as a solution for, among other things, minimizing these adverse consequences of polarization. Because the metal electrolyte interface in the gel electrode design is removed from the vicinity of the tissue, the Helmholtz layer is believed to be encapsulated in the gel. Since charge transport is by ionic movement in the gel, the ions can diffuse into and out of the gel freely and a charge layer is not established at the surface of the gel at which tissue contact is established. A large metal surface contacting the gel decreases the impedance of the Helmholtz layer. Also, the ion concentration can be increased by increasing available charge carriers, as in the case of 2-acrylamido-2-methylpropanesulfonic acid and salt polymers thereby reducing polarization and impedance even further. Improved sensing and lower stimulation thresholds result from the combined effects of repositioning of the Helmholtz layer and minimizing charge imbalances in the vicinity of the myocardial cells.

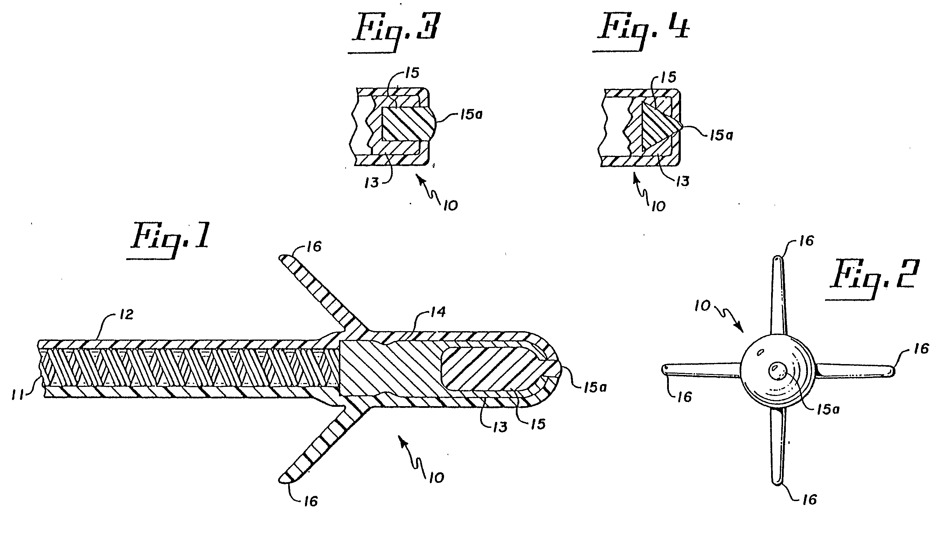

Referring now to Figs. 1 and 2, there is illustrated a body implantable lead constructed in accordance with the present invention. The lead body, designated 10, is comprised of an insulating plastic such as polyurethane and is attached to a coiled, flexible conductor 11 and an overlying sheath 12. Coiled, flexible conductor 11 may be of any known design and preferably has a central aperture which will accept a stylet (not shown) to provide stiffness during lead placement, in known manner. The lead body may also be non-linear such that stylet insertion will straighten the lead body to facilitate transvenous placement, also in known manner. To facilitate flexibility requirements as well as for reliability considerations, flexible conductor 11 may be a multi-filar member. Conductor 11 is in electrical communication with a polymer gel electrode 15 by means of a metal crimp-sleeve 14 and a metal contact electrode 13 defining a receptacle providing a large area of contact to polymer gel electrode 15 interior of lead body 10. Lead body 10 is sealed around electrode 15 so as to prevent the entrance of body fluids and the like. For example, it may be partially encapsulated in urethane polymer, mechanically interlocked or otherwise adhered thereto. A source of stimulation energy (not shown) for the delivery of stimulation energy to a desired body site is connected to conductor 11. Sheath 12 may be a preformed tubular member, preferably of a body compatible polyurethane the same as lead body 10, to overlie conductor 11 and provide electrical insulation therefor, in known manner. Also, as is known, lead body 10 may be tined as indicated at 16.

In the prior art, many lead bodies are formed of a molded silicone. A preferable material for sheath 12 and lead body 10 is a body-compatible polyurethane. Such polyurethanes meet the flexibility requirements of transvenous leads and are typically less thrombogenic than silicone and more resistant to tearing and cutting. In general, the physical characteristics of polyurethane are more suited to transvenous leads than those of silicone, although silicone or any other body compatible material may be employed in practicing the present invention.

Polymer-based gel electrode 15 may be provided in various configurations. It may be pre-cast or polymerized in situ, as desired.

In the embodiment shown, metal contact electrode 13 which may be of platinum, platinum-iridium, stainless steel, titanium or the like defines a cavity which contains polymer electrode 15 with a rounded end portion 15a thereof extending through the open distal end of the lead body. The end portion may also be flat. The rounded or flat end provides an exposed portion for establishing electrical contact between living tissue and the lead. It also provides a small surface area for tissue contact relative to its interior surface area and volume of contact with conductive receptacle 13. Such a configuration provides a low energy density at the interior contact surface and a high energy density at the tissue contact surface. A flat end portion will help focus the current through non-viable tissue to provide higher current density at the depolarizable myocardial level.

Figs. 3 and 4 illustrate other gel electrode configurations which provide a first portion inside body 10 having large area electrical contact with the electron source interiorly relative to the exposed second portion which provides a small area tissue contact.

A wide variety of lead configurations, including bipolar as well as unipolar, and polymer gel body configurations consistent with the tissue contacting requirements of the lead may be utilized.

Electrode 15 may consist of any conductive polymer gel. However, it preferably consists essentially of a hydrogel thick enough to allow space for the Helmholtz layer. Typically, thicknesses i.e., the dimension of electrode 15 extending out of the lead body, will range from about 0.5 mm to about 1.0 mm. Such an electrode body has been found to provide low mechanical trauma and high electrochemical interface with endocardial tissue.

Hydrogels for use with the invention are ionically conductive, soft, compliant, water swellable and inert to body tissues and fluids. Electrode 15 is also ionically conductive and permeable thereby establishing electrical communication between lead means 11 and the contacted tissue as well as providing a diffusion path to and away from the occluded tissue site.

Generally speaking, a hydrogel having the properties above described will comprise a coherent, three-dimensional polymeric network capable of imbibing water without dissolving. Usually, insolubility in water is provided by incorporating a crosslinked structure in the polymer. Hydrogels or water-containing gels may be comprised of water and various chemical substances including gelatin; polysaccharides; crosslinked acrylamide polymers; hydroxyethylmethacrylate polymers crosslinked polyhydroxyethylacrylate; polymerized, crosslinked 2-acrylamido-2-methylpropane sulfonic acid or one of its salts such as the sodium or potassium type; crosslinked polyvinylpyrrolidone; polyacrylic acid; copolymers of the aforementioned monomers with each other, and copolymers of the aforementioned monomers with other monomers such as styrene or other non-hydrogel forming monomers.

Hydrogels of low conductivity may be made conductive for purposes of this invention by incorporating salts such as sodium chloride, potassium chloride and like electrolytic salts into the hydrogel. This is most easily accomplished by dissolving a salt in a monomer-water-initiator solution and polymerizing. Other methods of preparation may also be used.

The specific preferred hydrogels are crosslinked polyacrylamide and crosslinked polymerized 2-acrylamido-2-methylpropane sulfonic acid or one of its salts. Volume 15, pages 273—291 of the *Encyclopedia of Polymer Science Technology* (1971), John Wiley Publishers, provides a section entitled HYDROGELS which describes the preparation of a variety of water-imbibing polymers. The content thereof is incorporated herein by reference.

Example — Polyacrylamide Hydrogel The following ingredients were dissolved in 52.5 grams of deionized water: 40.0 grams acrylamide, 2.0 grams sodium chloride, 8.0 cc of a 1% aqueous solution of methylene-bis-acrylamide. Nitrogen was vigorously bubbled through the resultant solution for 2.5 minutes. The following reagents were added to the solution simultaneously with stirring: 0.5 cc of a .38% solution of potassium bisulfite, 0.5 cc of a .38% solution of potassium persulphate, and 0.5 cc of a .24% solution of ferrous sulfate.

A portion of the resultant mixture was withdrawn into a syringe and ejected into the cavity of electrode tip 13 under a nitrogen atmosphere. The mixture was cured to form the hydrogel by allowing it to stand under the nitrogen atmosphere until solidified.

It is to be understood that, within the scope of the appended claims, the invention may be practiced otherwise than as specifically described.

Some of the subject matter disclosed herein is claimed in our European patent application nos. EP—A—57 450 and EP—A—57 451.

**Claims**

1. An implantable lead comprising a conductive polymer gel electrode (15) contacted over a relatively large part of its surface by a metal contact electrode (13) and having a relatively small part (15a) of its surface area exposed for contacting body tissue, the metal contact electrode (13) and the polymer gel electrode (15), except for the tissue-contacting surface thereof, being enclosed against contact with tissue and body fluids by an insulating sheath (12), and an electrical conductor (11) connected to the metal contact electrode (13).

2. The lead of claim 1 comprising a metal sleeve (14) providing for electrical communication between said metal contact electrode (13) and said electrical conductor (11).

3. The lead of claim 1 or 2 wherein said lead is of the tined transvenous type.

4. The lead of any of the preceding claims wherein said metal contact electrode (13) defines a cavity having an opening at the distal end thereof, and wherein said polymer gel electrode (15) is contained in said cavity with a relatively small portion thereof extending through the cavity opening for providing said exposed tissue contacting surface part (15a).

5. The lead of any of the preceding claims wherein the exposed tissue contacting surface part (15a) of the polymer gel electrode (15) defines a rounded configuration.

6. The lead of any of claims 1 to 4 wherein the exposed tissue contacting surface part (15a) of the polymer gel electrode (15) defines a flat configuration.

7. The lead of any of the preceding claims wherein the polymeric gel consists essentially of a hydrogel.

8. The lead of claim 7 wherein the hydrogel includes a crosslinked polymer.

9. The lead of claim 7 or 8 wherein the hydrogel includes a copolymer.

10. The lead of any of claims 7 to 9 wherein the hydrogel includes an acrylamide.

11. The lead of any of claims 7 to 9 wherein the hydrogel includes a hydroxyethylmethacrylate polymer.

12. The lead of claim 8 or 9 wherein the hydrogel includes a crosslinked ethylacrylate polymer.

13. The lead of claim 8 or 9 wherein the hydrogel includes a crosslinked polymer of 2-acrylamido-2-methylpropane sulfonic acid or one of its salts.

14. The lead of claim 8 or 9 wherein the hydrogel includes a crosslinked vinylpyrrolidone polymer.

15. The lead of claim 9 wherein the copolymer is comprised of at least two copolymerized hydrogel-forming monomers.

16. The lead of claim 9 wherein the copolymer is comprised of at least one hydrogel-forming monomer and one non-hydrogel-forming monomer copolymerized together.

17. The lead of any of the preceding claims wherein the polymeric gel contains relatively large ionic concentration relative to the tissue to be contacted.

## Patentansprüche

1. Implantierbare Leitung mit einer leitenden Polymergelelektrode (15), die über einen relativ großen Teil ihrer Oberfläche von einer Metallkontaktelektrode (13) kontaktiert ist und bei der ein relativ kleiner Teil (15a) ihrer Oberfläche freiliegt, um mit Körpergewebe in Kontakt zu kommen, wobei die Metallkontaktelektrode (13) und die Polymergelelektrode (15), mit Ausnahme des mit Gewebe in Kontakt kommenden Oberflächenteils dieser Elektrode, gegen Kontakt mit Gewebe und Körpermedien von einem isolierenden Mantel (12) umschlossen sind, sowie mit einem mit der Metallkontaktelektrode (13) verbundenen elektrischen Leiter (11).

2. Leitung nach Anspruch 1 mit einer Metallhülse (14), die für eine elektrische Verbindung zwischen der Metalkontaktelektrode (13) und dem elektrischen Leiter (11) sorgt.

3. Leitung nach Anspruch 1 oder 2, wobei die Leitung als mit Borsten versehene transvenöse Leitung ausgebildet ist.

4. Leitung nach einem der vorhergehenden Ansprüche, wobei die Metallkontaktelektrode (13) einen Hohlraum mit einer Öffnung an dem distalen Ende bildet, und wobei die Polymergelelektrode (15) in diesem Hohlraum untergebracht ist und ein relativ kleiner Teil dieser Elektrode durch die Hohlraumöffnung hindurchreicht, um den freiliegenden, mit Gewebe in Kontakt kommenden Oberflächenteil (15a) zu bilden.

5. Leitung nach einem der vorhergehenden Ansprüche, wobei der freiliegende, mit Gewebe in Kontakt kommende Oberflächenteil (15a) der Polymergelelektrode (15) eine abgerundete Gestalt hat.

6. Leitung nach einem der Ansprüche 1 bis 4, wobei der freiliegende, mit Gewebe in Kontakt kommende Oberflächenteil (15a) der Polymergelelektrode (15) eine flache Gestalt hat.

7. Leitung nach einem der vorhergehenden Ansprüche, wobei das Polymergel im wesentlichen aus einem Hydrogel besteht.

8. Leitung nach Anspruch 7, wobei das Hydrogel ein vernetztes Polymer aufweist.

9. Leitung nach Anspruch 7 oder 8, wobei das Hydrogel ein Kopolymer aufweist.

10. Leitung nach einem der Ansprüche 7 bis 9, wobei das Hydrogel ein Acrylamid aufweist.

11. Leitung nach einem der Ansprüche 7 bis 9, wobei das Hydrogel ein Hydroxyäthylmethacrylatpolymer aufweist.

12. Leitung nach Anspruch 8 oder 9, wobei das Hydrogel ein vernetztes Äthylacrylatpolymer aufweist.

13. Leitung nach Anspruch 8 oder 9, wobei das Hydrogel ein vernetztes Polymer aus 2-Acrylamid-2-methylpropan-sulfonsäure oder einem ihrer Salze aufweist.

14. Leitung nach Anspruch 8 oder 9, wobei das Hydrogel ein vernetztes Vinylpyrrolidonpolymer aufweist.

15. Leitung nach Anspruch 9, wobei das Kopolymer aus mindestens zwei kopolymerisierten, hydrogelbildenden Monomeren besteht.

16. Leitung nach Anspruch 9, wobei das Kopolymer aus mindestens einem hydrogelbildenden Monomer und einem nichthydrogelbildenden Monomer besteht, die zusammen kopolymerisiert sind.

17. Leitung nach einem der vorhergehenden Ansprüche, wobei das Polymergel eine gegenüber dem zu kontaktierenden Gewebe relativ große Ionenkonzentration aufweist.

## Revendications

1. Conducteur implantable caractérisé en ce qu'il comprend un électrode (15) de gel de polymère conducteur en contact par une partie relativement grande de sa surface avec une électrode de contact métallique (13) et comprenant une partie relativement petite (15a) de sa surface exposée pour être en contact avec un tissu du corps, l'électrode de contact métallique (13) et l'électrode de gel de polymère (15), sauf sa surface de contact avec un tissu, étant protégées contre tout contact avec les tissus et les fluides du corps par une gaine isolante (12), et un conducteur électrique (11) étant connecté à l'électrode de contact métallique (13).

2. Conducteur selon la revendication 1, caractérisé en ce qu'il comprend une *virole* métallique (14) assurant une communication électrique entre ladite électrode de contact métallique (13) et ledit conducteur électrique (11).

3. Conducteur selon la revendication 1 ou 2, caractérisé en ce que ledit conducteur est du type intraveineux à pointes.

4. Conducteur selon l'une quelconque des revendications précédentes, dans lequel ladite électrode de contact métallique (13) définit une cavité avec une ouverture à son extrémité distale, et dans lequel ladite électrode (15) de gel de polymère est enfermée dans ladite cavité avec une partie relativement réduite passant par l'ouverture de la cavité pour former ladite partie de surface (15a) exposée, en contact avec les tissus.

5. Conducteur selon l'une quelconque des revendications précédentes, dans lequel la partie de surface exposée (15a) en contact avec les tissus, de l'électrode (15) de gel de polymère, présente une configuration arrondie.

6. Conducteur selon l'une quelconque des revendications 1 à 4, dans lequel la partie de surface exposée (15a) en contact avec le tissu, de l'électrode (15) de gel de polymère, présente une configuration plate.

7. Conducteur selon l'une quelconque des revendications précédentes, dans lequel le gel de polymère consiste essentiellement en un hydrogel.

8. Conducteur selon la revendication 7, dans lequel l'hydrogel contient un polymère réticulée.

9. Conducteur selon la revendication 7 ou 8, dans lequel l'hydrogel contient un copolymère.

10. Conducteur selon l'une quelconque des revendications 7 à 9, dans lequel l'hydrogel contient une acrylamide.

11. Conducteur selon l'une quelconque des revendications 7 à 9, dans lequel l'hydrogel contient un polymère d'hydroxyéthylméthacrylate.

12. Conducteur selon la revendication 8 ou 9, dans lequel l'hydrogel contient un polymère d'éthylacrylate réticulé.

13. Conducteur selon la revendication 8 ou 9, dans lequel l'hydrogel contient un polymère réticulé d'acide sulfonique 2-acrylamido-2-méthylpropane ou l'un de ses sels.

14. Conducteur selon la revendication 8 ou 9, dans lequel l'hydrogel contient un polymère de vinyle pyrrolidone réticulé.

15. Conducteur selon la revendication 9, dans lequel le copolymère est constitué par au moins deux monomères formant un hydrogel copolymérisés.

16. Conducteur selon la revendication 9, caractérisé en ce que le copolymère est constitué par au moins un monomère formant un hydrogel et un monomère ne formant pas d'hydrogel, copolymérisés ensemble.

17. Conducteur selon l'une quelconque des revendications précédentes, dans lequel le gel de polymère contient une concentration ionique relativement grande par rapport au tissu en contact.

Fig.1  Fig.2  Fig.3  Fig.4